Europäisches Patentamt

European Patent Office

Office européen des brevets

(11) Publication number: 0 351 483 A1

# EUROPEAN PATENT APPLICATION

(21) Application number: 88830310.4

(22) Date of filing: 19.07.88

(51) Int. Cl.⁴: B65F 1/04 , A61B 19/02 , B65F 1/00 , B65F 7/00

(43) Date of publication of application:
24.01.90 Bulletin 90/04

(84) Designated Contracting States:
AT BE CH DE ES FR GB GR IT LI LU NL SE

(71) Applicant: Balestra, Alberto
Corso Regina Margherita 286
I-10143 Torino(IT)

(72) Inventor: Balestra, Alberto
Corso Regina Margherita 286
I-10143 Torino(IT)

(74) Representative: Notaro, Giancarlo et al
c/o Jacobacci-Casetta & Perani S.p.A. Via
Alfieri, 17
I-10121 Torino(IT)

(54) Container for hospital garbage.

(57) A container for hospital refuse is formed by a rigid outer casing (12), a flexible envelope (14) contained within the rigid outer casing (12), and an auxiliary rigid casing (16) situated within the flexible envelope (14) to prevent it from rupturing.

FIG.2

## A container for hospital refuse

The present invention relates to a container for hospital refuse, comprising a flexible envelope contained in a rigid casing.

The hospital refuse stored in such containers is often infected and, for the most part, is intended for incineration. After it has been introduced into the flexible container situated inside the rigid container, for example of cardboard, the refuse is normally treated with a disinfectant in order to prevent bacterial proliferation or putrefactive processes during the time interval which elapses between the filling of the container and its incineration, a time interval which may be of several days if not weeks.

In the containers used traditionally, it has been found that liquids escape from the flexible envelope as a result of rupture of the latter. In fact, sharp or pointed objects, such as syringe needles, removed prostheses or the like, may be present amongst the refuse. Moreover, the use of the disinfectant when the container has been filled may not be very easy for the operators and in some cases is neglected.

The object of the present invention is to provide a container of the type specified at the beginning of the description, which does not have the above problems and which is simple and cheap to produce.

According to the invention, this object is achieved by virtue of the fact that the container also includes an auxiliary rigid casing situated within the flexible envelope.

The flexible envelope preferably has an internal reservoir which is adapted to deliver a disinfectant liquid after filling and prior to closure of the container.

The refuse is thus placed inside the auxiliary rigid casing without any risk of rupture of the flexible envelope which is impermeable to both water and gases. Moreover, it is notably easier for the operator to disinfect the container, since it is only necessary to break a predisposed wall of the reservoir before the flexible envelope and the rigid outer casing are closed.

Further characteristic and advantages of the container according to the invention will become clear from the detailed description which follows with reference to the appended drawings, provided by way of non-limiting example, in which:

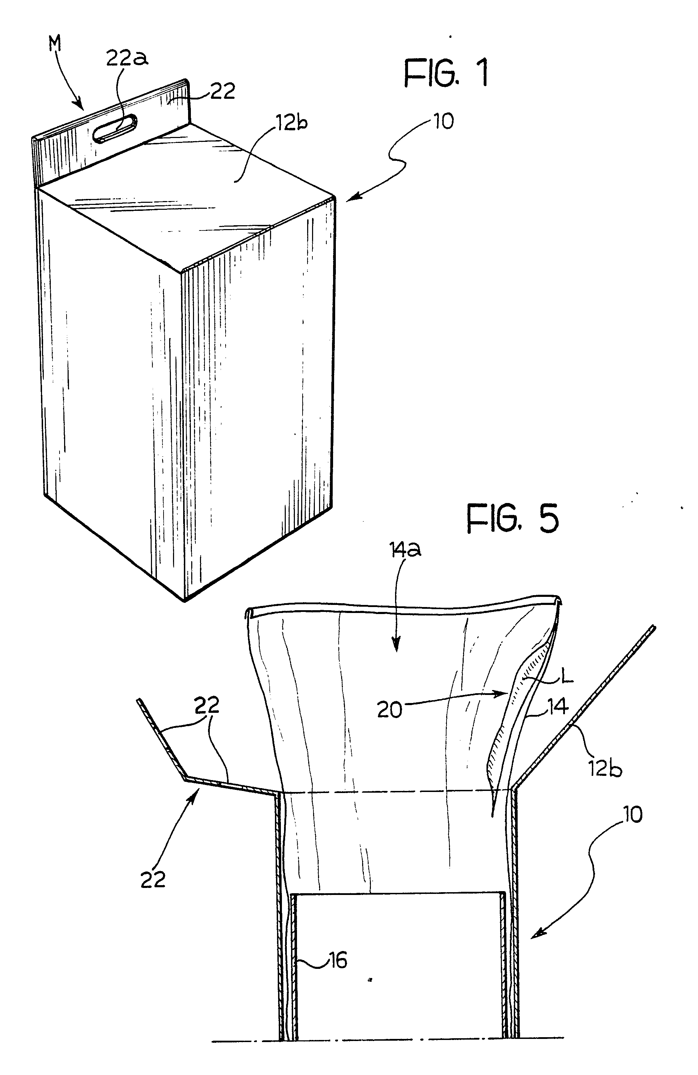

Figure 1 is a perspective view of a container according to the invention,

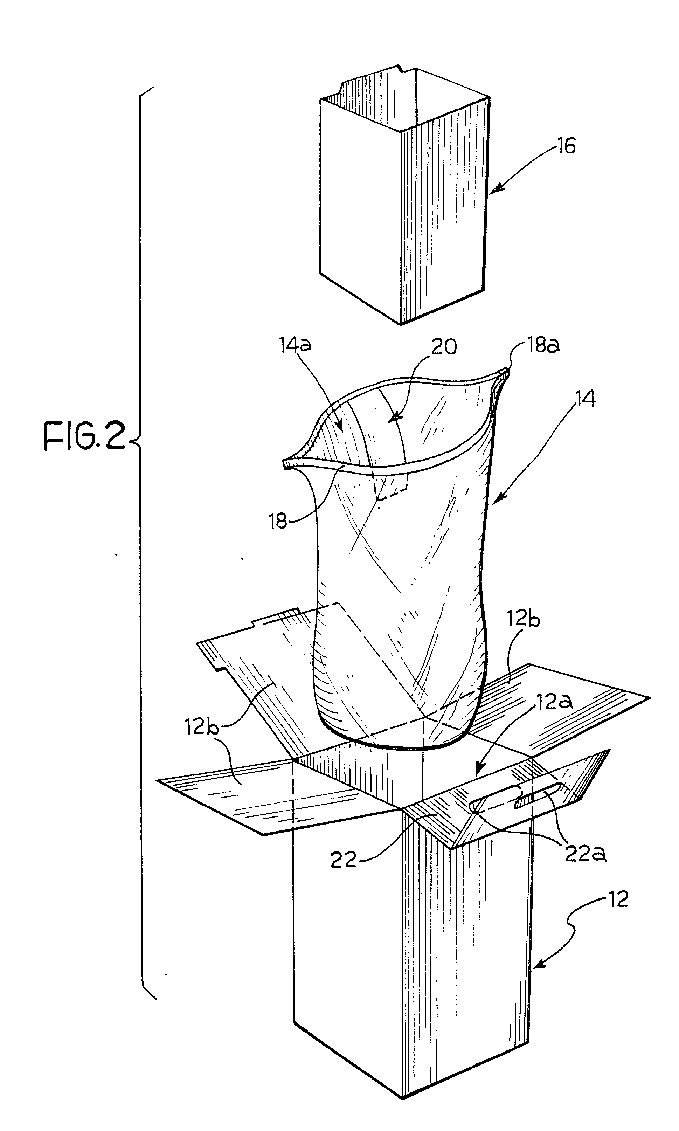

Figure 2 is an exploded perspective view of the container of Figure 1,

Figure 3 is a partial perspective view of the container in the open configuration,

Figure 4 is a perspective view similar to Figure 3 and shows the container in a partially closed configuration, and

Figure 5 is a section taken on the line V-V of Figure 3.

With reference to the drawings, a container for hospital refuse is generally indicated 10 and comprises a rigid, parallelepipedal outer casing 12 made of cardboard, and a flexible bag 14 of plastics material having a mouth 14a and arranged removably within the rigid casing 12.

An auxiliary rigid casing 16, which has slightly smaller dimensions in plan than the outer casing 12 and a smaller height than the latter, is inserted into the flexible bag 14 through the mouth 14a. To advantage, the auxiliary casing 16 is made from cardboard of a sufficient thickness to prevent pointed or sharp bodies introduced into it through the mouth 14a of the bag from perforating its side walls or base wall.

In correspondence with its mouth 14a, the flexible bag 14 has a rapid closure 18 of the type having interlockable edges and provided with a slider 18a; a flexible sachet 20 is fixed near one of the edges of the mouth 14a and contains a disinfectant liquid L.

In correspondence with its inlet opening 12a, the rigid outer casing 12 has foldable flaps 12b and a flap 22 which is bent into an L-shape and has apertures 22a adapted to act as a handle M for the picking up of the container 10.

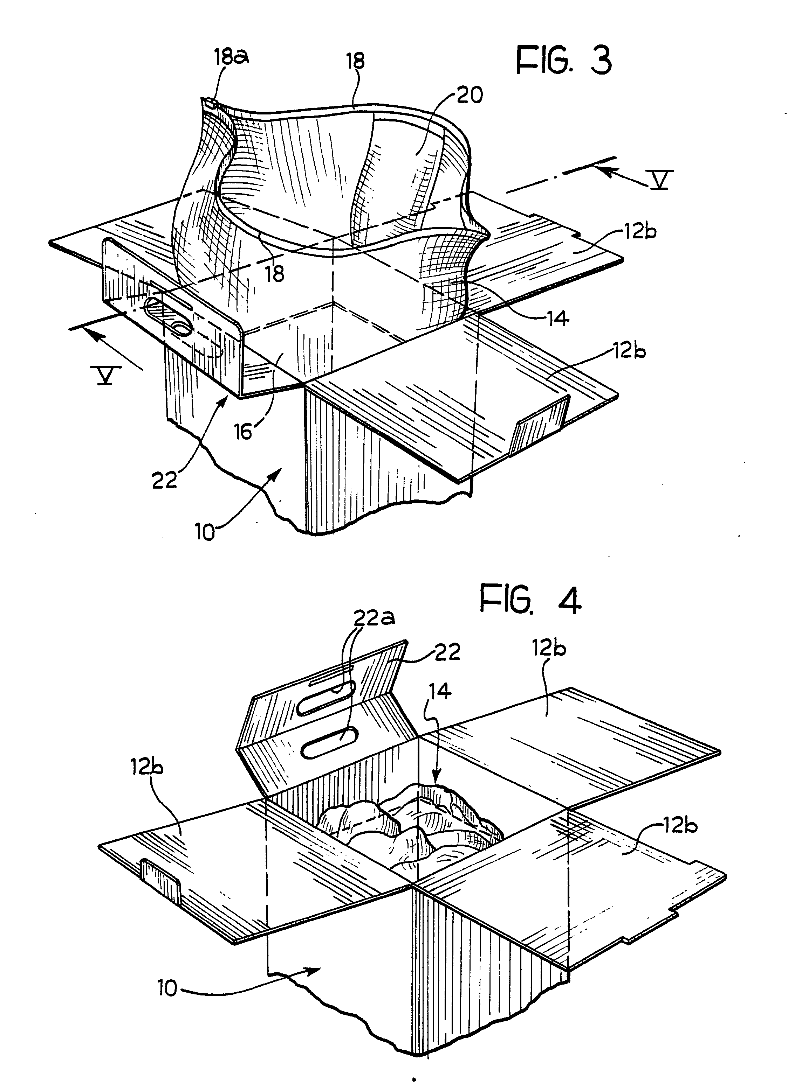

During the filling of the container, the latter is arranged as in Figure 3, that is, with the mouth 14a of the flexible bag open. When the auxiliary rigid casing 16 is full, the operator pierces the sachet 20 with any sharp object to cause the release of the disinfectant liquid L which falls onto the contents of the flexible bag 14. The mouth 14a of the flexible bag is then closed substantially hermetically by means of the slider 18a (the configuration shown in Figure 4) and the container 10 is closed by the folding over of the flaps 12b and the flap 22. The container can then face the journey to the incinerator without any danger of liquid escaping from the flexible bag 14 and hence without any danger of contamination.

According to another embodiment (not illustrated in the drawings), the rigid outer casing and the auxiliary casing are formed with bellows-like side walls so as to enable the container to be squashed once it is filled, in order to reduce the space occupied by the container itself during transport.

## Claims

1. A container for hospital refuse, comprising a flexible envelope contained in a rigid casing, characterised in that it also includes an auxiliary rigid casing (16) situated within the flexible envelope (14).

2. A container according to Claim 1, characterised in that the flexible envelope (14) has an internal reservoir (20) which is adapted to deliver a disinfectant liquid (1) after filling and prior to closure of the container (10).

3. A container according to Claim 1, in which the flexible envelope is constituted by a bag (14) of plastics material having a mouth (14a) for the introduction of the refuse, characterised in that the reservoir is constituted by a flexible sachet (20) of plastics material connected to the inside of the bag (14) in correspondence with its mouth (14a) and adapted to be pierced at the time of use.

M

22a

22

12b

10

FIG. 1

FIG. 5

14a

20

L

14

22

22

22

12b

10

16

FIG.2

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DE-U-8 608 885 (R.W.G.K.)<br>* Page 4, line 16 - page 5, line 9;<br>page 9, lines 8-12; page 10, line 21 -<br>page 11, line 8; figures 4-9 *<br>--- | 1 | B 65 F 1/04<br>A 61 B 19/02<br>B 65 F 1/00<br>B 65 F 7/00 |
| Y | DE-C- 109 538 (MASCHKE)<br>* Page 1, column 2, line 11 - page 2,<br>column 2, line 22; figures *<br>--- | 1 | |
| A | US-A-1 421 628 (WATKINS)<br>* Page 1, lines 40-83; figures 1-3 *<br>--- | 1 | |
| A | FR-A-1 475 399 (ANDERSEN)<br>* Page 2, column 2, lines 12-30;<br>figures *<br>--- | 1-3 | |
| A | FR-A-1 570 665 (CANNON)<br>* Page 2, line 25 - page 3, line 6;<br>figures 2,3 *<br>--- | 2,3 | |
| A | DE-U-8 707 533 (FABRITZ)<br>--- | | TECHNICAL FIELDS<br>SEARCHED (Int. Cl.5) |
| A | DE-A-3 317 300 (VIEREGGE-BRUHNS)<br>--- | | A 61 B<br>B 65 F<br>A 61 M |
| A | DE-U-8 804 445 (W.D.K.)<br>--- | | |
| A | DE-A-3 321 603 (BARKEY)<br>----- | | |

The present search report has been drawn up for all claims

| Place of search | Date of completion of the search | Examiner |
|---|---|---|
| THE HAGUE | 28-08-1989 | KLEIN C. |